(19) Europäisches Patentamt
European Patent Office
Office européen des brevets

(11) EP 2 584 971 B1

(12) EUROPEAN PATENT SPECIFICATION

(45) Date of publication and mention
of the grant of the patent:
**10.11.2021 Bulletin 2021/45**

(21) Application number: **11798920.2**

(22) Date of filing: **23.06.2011**

(51) Int Cl.:
$G01S\ 7/52^{(2006.01)}$

(86) International application number:
**PCT/US2011/041625**

(87) International publication number:
**WO 2011/163475 (29.12.2011 Gazette 2011/52)**

(54) **ULTRASOUND IMAGING WITH ANALOG PROCESSING**

ULTRASCHALLBILDGEBUNG MIT ANALOGVERARBEITUNG

SYSTÈME D'IMAGERIE ULTRASONORE AVEC TRAITEMENT ANALOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2010 US 357819 P**
**18.08.2010 US 374946 P**

(43) Date of publication of application:
**01.05.2013 Bulletin 2013/18**

(73) Proprietor: **Analog Devices, Inc.**
**Wilmington, MA 01887 (US)**

(72) Inventors:
• **VIGODA, Benjamin**
**Winchester**
**Massachusetts 01890 (US)**
• **BERNSTEIN, Jeffrey**
**Middleton**
**Massachusetts 01949 (US)**
• **NESTLER, Eric**
**Concord**
**Massachusetts 01742 (US)**

(74) Representative: **Yang, Shu et al**
**Withers & Rogers LLP**
**2 London Bridge**
**London SE1 9RA (GB)**

(56) References cited:
| WO-A1-02/17298 | US-A- 4 173 007 |
| US-A- 5 469 851 | US-A- 5 676 147 |
| US-A1- 2005 261 596 | US-A1- 2007 239 001 |
| US-B1- 6 193 659 | US-B2- 7 110 556 |
| US-B2- 7 164 768 | |

• SILVA MARTINEZ J ET AL: "Discrete time filters", WILEY ENCYCLOPEDIA OF ELECTRICAL AND ELECTRONICS ENGINEERING, XX, XX, 27 December 1999 (1999-12-27), pages 631-643, XP002584894, DOI: 10.1002/047134608X.W2238
• CHANG-HONG HU ET AL: "Development of a real-time, high-frequency ultrasound digital beamformer for high-frequency linear array transducers", IEEE TRANSACTIONS ON ULTRASONICS, FERROELECTRICS AND FREQUENCY CONTROL, IEEE, US, vol. 53, no. 2, 1 February 2006 (2006-02-01), pages 317-323, XP011148762, ISSN: 0885-3010, DOI: 10.1109/TUFFC.2006.1593370

## Description

## BACKGROUND

This document relates to ultrasound imaging with analog processing.

Imaging systems, for instance medical ultrasound imaging systems, often perform signal processing of acquired signals for purposes such as beam forming. In some examples, initial processing may be performed in a probe in which sensor signals are digitized and then processed using digital signal processing techniques before being passed to another component of the system in which images are formed from the processed signals.

In a ultrasound system in which the transducers in a probe form an array, one approach to sending excitation ultrasound signals and reconstructing images of tissue within the body from received ultrasound signals is to transmit excitation signals for each of the transducers from a main processing unit to the probe and then to transmit the received signals from the transducers back to the main processing unit. In some examples, each transducer has a separate electrical conductor for passing a signal to and/or from the main unit. In some examples, various forms of multiplexing or modulation are used to reduce the number of conductors required to pass the signals between the main unit and the probe. Processing the signals includes introducing desired delays on signals associated with different transducers to focus the delivery of transmitted ultrasonic signals or to focus the reception of signals on selected locations within the body.

In some ultrasound systems, some degree of processing is performed in the probe, thereby reducing the number of conductors needed for communication between the probe and a main unit or to reduce the amount of information passed between the probe and the mail unit. One form of processing is to introduce delays for input or output signals for different transducers to vary the focus of signals within the body. In some examples, a memory and a clocked system is used to sample signal to introduce delays that are an integral number of sampling periods by storing input or output values in the memory and retrieving them at the desired delay. In some examples, delay is introduced using a configurable analog phase delay element. In some examples, subsets of transducers form "micro-arrays" and suitably delayed received signals are added together to form a combined signal such that each microarray has a single conductor or channel linking the probe and the main unit.

There is a need to perform more processing of the transmitted and/or received signals in an ultrasound probe in order to reduce the communication requirements between the probe and a main unit, or to completely eliminate the main unit entirely to form a portable imaging system, for example, with an integrated display.

There is also a need to perform processing using reduced power in a wireless probe, thereby permitting longer operating duration between battery charges or to reduce the power delivery requirements from a main unit to the probe or to reduce the power dissipation requirements of the probe. Also, in a two part system with a probe and a main unit, there is a need to reduce the amount of information passing from the probe to the main unit, thereby reducing the bandwidth requirement (e.g., over a wireless link) and/or transmission power requirement of the probe.

Patent Publication No. WO0217298 (A1) describes an ultrasonic imaging method and apparatus for imaging a volumetric region using a two dimensional array transducer. The ultrasonic probe includes the 2D array transducer and microbeamformers for beamforming signals from groups of elements of the array in the probe.

Patent Publication No. US4173007 (A) describes a dynamically variable electronic delay line for real time ultrasonic imaging systems, so as to controllably phase the signals associated with an array of electromechanical transducer elements and thereby enable the selective scanning and dynamic focusing of a target.

Patent Publication No. US5676147 (A) describes an ultrasonic receive beamformer with phased sub-arrays. Here, an ultrasonic receive beamformer includes transducers forming receive signals that are applied to sub-array processors.

Chang-Hong Hu et al, "Development of a Real-Time, High-Frequency Ultrasound Digital Beamformer for High-Frequency Linear Array Transducers", IEEE Transactions on Ultrasonics, Ferroelectrics and Frequency control, IEEE, vol. 53, no. 2 (2006) describes a real-time digital beamformer for highfrequency (20 MHz) linear ultrasonic arrays. Radio frequency (RF) signals are digitized, delayed, and summed through a real-time digital beamformer, which is implemented using a field programmable gate array (FPGA). Using fractional delay filters, fine delays as small as 2 ns can be implemented.

Patent Publication No. US6193659 (B1) describes a medical ultrasonic diagnostic imaging method and apparatus. Here, an improvement to the method for harmonic imaging including the steps of (a) transmitting ultrasonic energy at a fundamental frequency and (b) receiving reflected ultrasonic energy at a harmonic of the fundamental frequency.

Patent Publication No. US5469851 (A) describes a phased array digital ultrasound beamformer for use with an ultrasound transducer array. The beamformer includes a processing channel for each element of the transducer array. Each processing channel includes a digitizing circuit for converting the received signal to digital samples and a time multiplexed digital delay circuit responsive to delay coefficients for delaying the digital samples by time multiplexed delays to produce delayed, time multiplexed samples for forming two or more receive beams.

## SUMMARY

In one aspect, in general, an approach to signal processing in an imaging system, for instance an ultrasound medical imaging system, makes use of analog signal processing prior to conversion to digital signals. In some examples, the outputs of ultrasound signals are acquired in analog form and processed in a discrete time analog circuit, for example, using techniques described in the co-pending application titled "ANALOG COMPUTATION," referenced above.

According to an aspect of the present invention, there is provided an ultrasound processing system in accordance with claim 1.

According to another aspect of the present invention, there is provided a method in accordance with claim 12.

Aspects can include one or more of the following features.

The ultrasound processing system further comprises a plurality of ultrasound elements, each coupled to provide a signal representing an ultrasound signal received at the ultrasound element to a corresponding input processing block.

The ultrasound processing system further comprises a plurality of amplifiers, each coupled to an input of a corresponding input processing block. In some examples, at least some of the processing blocks are passive (i.e., without signal amplification elements).

Each input processing block comprises a passive charge sharing processing section including a plurality of capacitive elements and a plurality of switches (e.g., transistors) for controlling transfer of charge between the capacitive elements, wherein characteristics of the processing section are controllable according to sequencing of operation of the switches.

A controller is coupled to the input processing blocks for controlling operation of one or more of the input processing blocks.

Each input processing block further comprises a coarse delay stage controllable to introduce a delay that is a multiple of the sampling period.

The ultrasound processing system further comprises an analog-to-digital converter (ADC) coupled to an output of each of (or at least some of) the processing stages.

The ultrasound processing system further comprises combination circuitry for combining multiple outputs of the input processing blocks to form a combined signal.

The ultrasound processing system further comprises an analog-to-digital converter (ADC) coupled to an output of the combination circuitry.

The probe is linked to a base unit by a communication link suitable for passing the combined signals to the base unit.

The input processing blocks and the combination circuitry are within a portable probe of the system.

The ultrasound processing system further comprises a controller coupled to the input processing blocks for repeatedly reconfiguring the input processing blocks.

The controller is responsive to the combined signals to adapt to signal acquisition characteristics of the input signals.

Each input processing block comprises a plurality of a passive signal scaling circuits each for accepting an analog input signal value and a digital scaling control value representing a scaling factor and storing an analog representation of a scaled signal value equal to a product of the accepted signal value and the scaling factor in an output stage for the scaling circuit.

Each signal scaling circuit comprises a plurality of switchably interconnected capacitive elements

In operation of the scaling circuit, the scaled signal value is formed in a succession phases, each phase being associated with a configuration of the switchable interconnection of capacitive elements permitting charge sharing among interconnected capacitors, at least one of the capacitive elements being configured according to the digital scaling control value.

The ultrasound processing system further comprises a plurality of controllable output processing blocks for generating a corresponding signal for emission as an ultrasound signal, each block implementing a discrete time analog signal processing stage, and controllable to introduce relative delay between signals that are a fraction of the sampling period of the signal processing stage.

Advantages of one or more aspects of the system can include reduced power requirements for the processing in a probe, which may enable wireless battery-powered operation and which may reduce the size, complexity, or component costs of the probe.

Increased processing in the probe can reduce communication capacity required between a probe and a base unit, thereby reducing cost and complexity of a two-part ultrasound system.

Other advantages can include improved imaging performance by enabling more complex processing (e.g., in the probe) than is feasible using purely digital signal processing techniques.

Other aspects, features, and advantages are apparent from the following description and from the claims.

Description of Drawings

FIG. 1 is a block diagram of an ultrasound imaging system;

FIG. 2 is a block diagram showing signal processing blocks; and

FIG. 3 is a block diagram of a signal processing unit for use in an ultrasound imaging system.

## Description

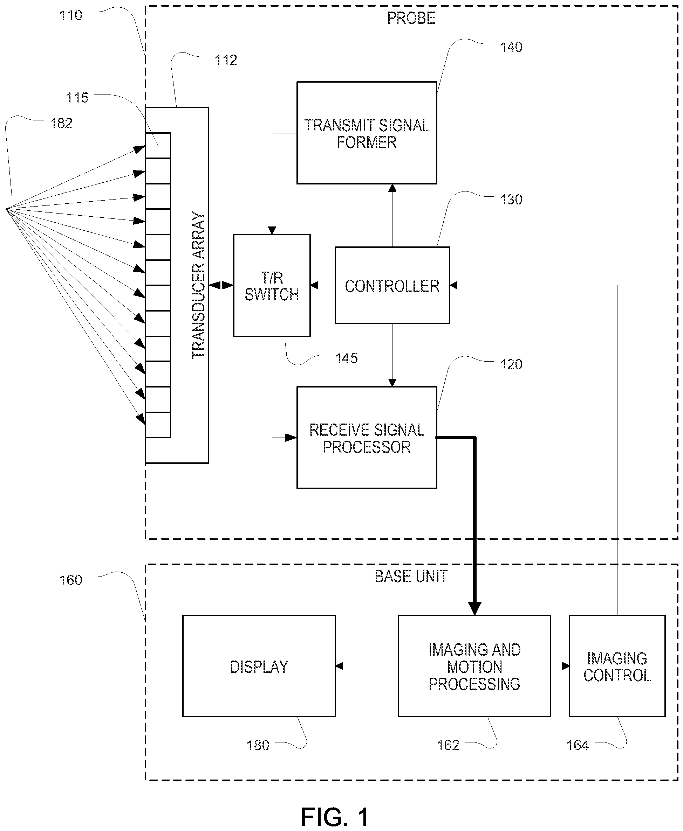

Referring to FIG. 1, an example of an ultrasound system 100 includes a probe 110 and a base unit 160 coupled to the probe via a communication link. In other examples, the system is portable, and some or all of the elements of the base unit are hosted within the probe itself.

The ultrasound system makes use of an array 112 with a set of ultrasonic elements 115, for example 256 or 1024 or more arranged in a linear or grid pattern. These elements are used to emit and sense ultrasonic signals. These emitted signals are reflected within the patient's body and the reflected signals are sensed at the ultrasonic elements. A transmit signal former 140 generates the signals for transmission from the elements, and a receive signal processor 120 processes the sensed signals. A transmit/receive switch circuit 145 is used to alternate between transmission and receiving phases of operation.

A beamforming approach is used in which the ultrasonic signals emitted from the elements are formed to create a focused signal at one or more desired locations within the body being sensed. Similarly, the signals received at the ultrasonic elements are processed in order to selectively acquire reflections originating at desired locations within the body.

Generally, the receive signal processor 120 performs some or all of its processing in an analog domain prior to performing analog-to-digital conversion (ADC) of the received signals at the probe or base unit. In some examples, some of this analog processing is performed prior to amplification and reduces the performance requirements of such amplifiers. The system may perform one or more of the following analog domain signal processing steps prior to digitization:

- Time delay

- Anti-alias filtering

- Matched filtering (e.g., for processing of coded excitation signals)

- Gain control

- Transform (e.g., Fourier, compressing sensing) analysis

- Matrix operations

Similarly, the formation of the excitation signals in the transmit signal former 140 may make use of analog processing techniques, for example, to introduce delays suitable for focusing the emitted signals on desired parts of the body.

Generally, the processing performed by the receive signal processor 120 and/or the transmit signal former 140 varies as an image is acquired, for example, to perform a scanning operation in which a focus of the emitted signals is scanned through a three-dimensional body volume and the acquired signal is similarly focused to different locations in the body volume. A controller 130 sends control signals to the processor 120 and former 140. For example, control signal may encode desired delays to introduce in the various output or input signal paths, or may provide more specific processing characteristics, for example, parameters of filters.

The analog processing implemented in the transmit signal former 140 and/or the receive signal processor 120 make use of discrete time analog domain processing in which capacitors are coupled by controlled switches in order to accomplish desired signal processing functions by successive transfers of charge between the capacitors. A number of signal processing techniques using such charge sharing approaches are described in one or more of the following applications:

- U.S. Patent Publication No. US2010/0207644A1 on August 19, 2010, titled "ANALOG COMPUTATION".

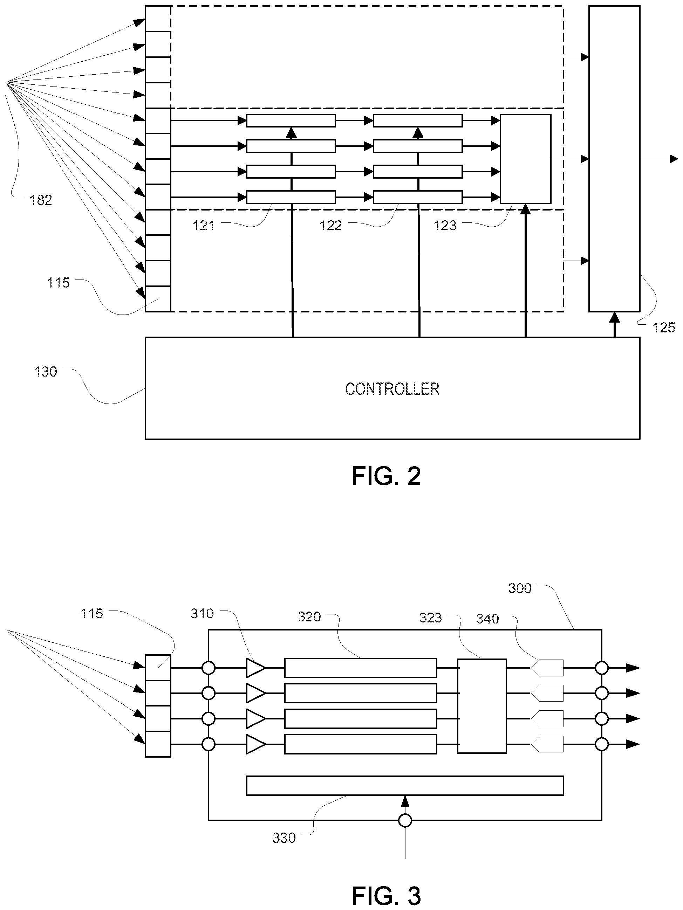

Referring to FIG. 2, in one example, each ultrasound element 115 provides an analog signal that is sampled at a rate that is sufficiently high to avoid aliasing, for example, based on a band-limit of the sensors and/or an anti-aliasing filter between the sensor and the sampling unit (not shown). Each signal is processed to introduce a delay controlled by the controller 130 in order to focus the acquired signal from a desired point 182 in the body. According to the invention, each signal passes through two delay stages. A coarse delay stage 121 introduces a delay that is an integral number of sampling periods, for example using a capacitor array provides the temporary storage of the analog signal values. A second stage 122 provides a fine time delay, which is less than a full sampling period. According to the invention, this second stage 122 is implemented using a time domain filter that provides an appropriate interpolation of sample values in order to effect the desired delay.

Fractional delay is implemented using a Finite Impulse Response (FIR) filter, which can be expressed as

$$y(n) = \sum_{k=0}^{K-1} h_k\, x(n-k).$$

The coefficients $h_k$ are chosen, for example, using a windowed fractionally offset sinc function. In one implementation, each fractional delay stage 122 includes $K^2$ capacitors (doubly indexed (0,0) through ($K-1,K-1$)). An input $x(i)$ output from the coarse delay at time

$i$ is coupled to multiple capacitors ($i$ mod $K$, 0)..., ($i$ mod $K, K-1$) such that they are all charged based on the same input. Then, an output y(i) is formed by coupling capacitors ($i$ mod $K$, 0), ($i-1$ mod $K$,1), ... , ($i-K+1$ mod $K, K-1$), optionally with an output capacitor or a capacitor in another storage section. In order to accommodate different fractional delays, different sets of filter tap values may be achieved by making the (j,k)th capacitor value $c_{(j,k)}$ have a selectable value that is proportional to $h_k$. For example, if a fixed number of fractional delay values can be selected by the controller, a different set of $K^2$ capacitors can be selected for each different fractional delay.

Rather than having $K^2$ fixed capacitors for each possible delay, the capacitor values may be controllable, for example, according to a binary control value to selects a parallel combination of power-of-2 sized capacitors.

Other approaches to FIR filter implementations that have many preferable characteristics are those in which the effective capacitance that is used to implement the filter tap values have multiple controlled capacitors and/or use multiple phases of charge sharing. An advantage of such configurations is that a relatively large range of capacitance and therefore coefficient values can be achieved with a relatively small total capacitance (which can reduce circuit area), while providing relatively high precision in the selectable values. In one example of an FIR filter that uses this approach, $K^2$ fixed sampling capacitors are used as described above (e.g., each having the same value), and each FIR output is formed in a number sharing phases, for example, in a one or more phases in which each of $K$ sampling capacitors is coupled to a digitally controlled capacitor or capacitor network to transfer a selectable amount of charge from the sampling capacitor, and a last phase in which a subset of the capacitors are coupled together effectively computing a weighted average, to form the filter output value represented as a voltage or as a charge on an output capacitor.

The controller 130 configures the filters according to the desired delays, for example, by specifying the filter coefficients for the finite impulse response filters, in order to focus the input signals on successive points 182. Note that as the body is scanned, the controller successively reconfigures the filters in order to scan various locations in the body.

After introducing the delay into the input signals, multiple signals are combined in a combiner 123, for example, by summing or averaging (e.g., by charge sharing) the signals. In some examples, a controllable gain is introduced to account for sensitivity variations of the different ultrasonic elements and/or to account for different attenuation along the paths to each of the elements through the body. In some examples, the combiner 123 also performs an envelope detection function on the combined signal.

In some examples, as shown in FIG. 2, the array of ultrasonic sensors is divided into a set of groups. Each group is processed as described above introducing controlled delays and optionally gains. A further processing stage 125 is optionally applied to multiple of the groups, for example, to all of the groups in the array. In some examples this processing is performed in the (discrete time) analog domain, while in other embodiments, an analog-to-digital conversion is performed prior to the further processing stages, which is implemented using digital signal processing. In some embodiments, the further processing can include one or more of spatial domain and transform domain processing.

Turning back to FIG. 1, the transmission of signal information from the receive signal processor to the image and motion processing module 162 at the base unit may be analog or digital. For example, the analog signal may comprise a beamformed and spatially subsampled analog signal, which requires substantially fewer analog signal paths (e.g., micro-coaxial cables) between the probe and the base unit than conventional approaches. In other examples, signals are digitized after processing at the probe and are transmitted to the base unit in digital form, for example, time multiplexed on a serial communication link, for example, over a wireless communication domain.

Note that an analog-to-digital converter (ADC) can sample the envelope at a slower rate than that used in the filtering stages, and the filtering stages can implement an anti-aliasing filter to prevent aliasing by using the lower sampling rate.

The base unit processes the received digitized signals in an imaging and motion processing module 162, which provides the synthesized image on a display 180 to a user of the system. An image controller also receives the signals, can controls aspects including overall control of the raster process in the probe.

In some versions of the system, an imaging control 164 performs more complex adaptations, for example, to account for various signal propagation factors that may degrade the signal. For example, the required delays, pulse waveforms, etc. introduced on the transmit and receive paths may be adjusted to account for propagation rate, dispersion, etc. of the signals before reaching the sensors.

In some examples, the analog filters described above in the context of providing fractional delay also (or instead) perform a matched filter function to improve detection of the reflected pulses, or for pulse compression and/or use of coded or pseudo-random excitation waveforms.

In some examples, the processing for each group of sensors, as well as the ADC for that block are integrated into a single electronic circuit or package (referred to below as a unit), as illustrated in FIG. 3. In an example of such a unit, a set of sensor inputs is provided as analog signals, illustrated as four separate inputs in the figure, understanding that other examples, may have different numbers of inputs (e.g., 8, 14 for a 4x4 patch, etc.). The unit optionally includes analog signal amplification elements 310, whose gain is optionally controllable by a controller 330 in the unit. The unit includes a number signal processing blocks 320, 323. In some examples, a separate signal processing block 320 is associated with each input, and another signal processing block 323 is used for combination of the signals. It should be understood that some versions of such a unit may have only the signal specific blocks 320 or only a combined processing block 323. In some versions of the unit, each signal processing block 320 performs a discrete (sampled) time analog signal domain processing of the signal, for example, using switched capacitor techniques as described above. The controller 330 is coupled to the processing blocks 320 to coordinate operation of the switches in the block in order to cause the desired sequence of charge sharing to implement desired signal processing functions, such as fractional sampling period delay, matched filtering, etc. In some versions of the system, the entire processing block 320 is passive in that gain is not introduced into the signal path through the block (recognizing that the switches in the block may be implemented using transistors, which are active devices, but the block nevertheless is passive with respect to the signal path). In some versions of the unit, further signal amplification may be introduced in the signal path, for example, between the signal blocks 320 and the combination signal block 323. In some versions of the system, the combination block is controllable to perform a sum or average of the ouputs of the processing blocks 320, and an envelope detection (e.g., rectification and smoothing). In some versions of the unit, analog outputs are provided from the unit, while in units as illustrated in FIG. 3, one or more analog-to-digital converters 340 process output of the processing block 323. Note that as illustrated the combination block 323 has the same number of inputs as outputs, but it should be understood that in some versions, the block performs a reduction in the number of signals. For example, there may be four inputs and only one output, which can be configured to provide a beamformed combination of the inputs. In some implementations, multiple of such units are integrated together in one circuit or package to process all the signals provided by the ultrasonic elements 115 of a probe array.

Note that the description above focuses on receiving ultrasound signals. The transmit portion of the probe (e.g., the transmit signal former 140) can be similarly configured, with each of multiple transducer outputs being driven by a corresponding signal that is appropriately delayed a fractional amount in a discrete time analog processing stage in order to focus the transmitted signals on the point in the body being images.

It should be understood that although the description above may focus on use of the analog signal processing to implement delay-based beamforming, other embodiments may perform other types of signal processing in the analog domain. For example, the signal processing blocks may perform joint processing, effectively implementing a multidimensional signal processing approach using the discrete time analog signal processing techniques referred to above.

As one example a transform approach may be used. For example, an analog implementation of a Discrete Cosine Transform (DCT) can be used to encode segments of the sensor grid (e.g., prior to or after performing delay based processing). In some implementations, a DCT or other transform may be used to compress the signal to reduce the amount of information that needs to be transmitted from or stored at the probe.

More generally, it should be recognized that the analog processing approaches described above and in the co-pending applications can be used to implement matrix multiplication operations. In some embodiments, the probe is configurable to perform such operations at the command of the controller at the probe and/or at the command of the base unit. Examples of functions that may be implemented in such a matrix form include transforms (e.g., effectively using square matrices) and projections (e.g., using rectangular matrices). As an example, a compressive sensing approach may be used in which a high number of sensor values are projected into a lower dimensional signal that is processed or transmitted for further processing. In some examples, the matrix operations are performed in stages, for example, combining subsets (e.g., patches) of sensors in a first matrix operation, and then combining the outputs across multiple subsets in successive stages. In some examples, a configurable probe can provide conventional beamforming functions are well as more complex multidimensional operations on the sensed signals.

In some examples, the processing in the main section provides control and/or feedback signals to configure or control the analog processing in the probe. For instance, the feedback may provide updated projection matrices for compressive sensing applications, gain control, and beam forming pattern. This feedback may be based, for example, on predicted characteristics of the sensed signals, which may be based on estimates of motion of the probe or body being sensed.

In some examples, the analog processing may include probabilistic computation, for instance, using model based or Bayesian approaches. The processing (e.g., at the base unit of the system) may include optimization-based reconstruction of an image, and output of signals suitable to driving an image presentation to a user of the system.

As introduced above, the analog processing may all be performed in a probe, and optionally digitized before being transmitted using a wired or wireless link to a main section of the system. The analog and/or digital processing may be controlled by software stored in a computer-readable for controlling a processor, such as a digital signal processor or general purpose computer. In some examples, software controlling the probe, including controlling the operation of the analog domain filters and transformation stages, is uploaded to the probe from the base unit, and stored in a tangible storage medium at the probe.

It is to be understood that the foregoing description is intended to illustrate and not to limit the scope of the invention, which is defined by the scope of the appended claims. Other embodiments are within the scope of the following claims

**Claims**

1. An ultrasound processing system comprising:

a plurality of controllable input processing blocks ( 320), each implementing a discrete time analog signal processing stage for processing one or more corresponding input signals representing ultrasound signals, wherein each input processing block comprises:
a first delay stage (121), controllable to introduce relative delays between signals, wherein the relative delays are a multiple of the sampling period of the signal processing stage; and
a second delay stage (122), controllable to introduce relative delays between signals, wherein the relative delays are a fraction of the sampling period of the signal processing stage;
wherein, for each input processing block:

the fractional delays of the second delay stage are implemented using a Finite Impulse Response, FIR, filter comprising a plurality of capacitive elements and a plurality of switches for controlling transfer of charge between the capacitive elements;
an input of the second delay stage is coupled to an output of the first delay stage, such that the plurality of capacitive elements is charged based on the output of the first delay stage; and
characteristics of the FIR filter are controllable according to sequencing of operation of the switches.

2. The ultrasound processing system of claim 1 further comprising one or more of:

a) a plurality of ultrasound elements (115), each coupled to provide a signal representing an ultrasound signal received at the ultrasound element to a corresponding input processing block;
b) a plurality of amplifiers (310), each coupled to an input of a corresponding input processing block (320);
c) an analog-to-digital converter (340) coupled to an output of each of the processing stages;
d) a plurality of controllable output processing blocks for generating a corresponding signal for emission as an ultrasound signal, each block implementing a discrete time analog signal processing stage, and controllable to introduce relative delay between signals that are a fraction of the sampling period of the signal processing stage.

3. The ultrasound processing system of claim 2 further comprising a controller (130, 330) coupled to the input processing blocks for controlling operation of one or more of the input processing blocks (320).

4. The ultrasound processing system of claim 1 further comprising: combination circuitry (123, 323) for combining multiple outputs of the input processing blocks to form a combined signal.

5. The ultrasound processing system of claim 4 wherein one or more of the following apply:

a) the system further comprises an analog-to-digital converter, ADC, coupled to an output of the combination circuitry;
b) the probe is linked to a base unit by a communication link suitable for passing the combined signals to the base unit.

6. The ultrasound system of claim 4 wherein the input processing blocks ( 320) and the combination circuitry (123, 323) are within a portable probe of the system.

7. The ultrasound system of claim 1 further comprising a controller (130, 330) coupled to the input processing blocks for repeatedly reconfiguring the input processing blocks.

8. The ultrasound system of claim 7 further comprising: combination circuitry (123, 323) for combining multiple outputs of the input processing blocks to form a combined signal wherein the controller (130, 330) is responsive to the combined signals to adapt to signal acquisition characteristics of the input signals.

9. The ultrasound system of claim 1, wherein each input processing block ( 320) comprises:
a plurality of a passive signal scaling circuits each for accepting an analog input signal value and a digital scaling control value representing a scaling factor and storing an analog representation of a scaled signal value equal to a product of the accepted signal value and the scaling factor in an output stage for the scaling circuit.

10. The ultrasound system of claim 9, wherein each signal scaling circuit comprises a plurality of switchably interconnected capacitive elements.

11. The ultrasound system of claim 10, wherein in operation of the scaling circuit, the scaled signal value is formed in a succession phases, each phase being associated with a configuration of the switchable interconnection of capacitive elements permitting charge sharing among interconnected capacitors, at least one of the capacitive elements being configured according to the digital scaling control value.

12. A method for processing ultrasound generated signals comprising:

accepting a plurality of a plurality of ultrasound generated signals;
processing the signals in a plurality of controllable input processing blocks ( 320), including performing a discrete time analog signal processing of the signals;
controlling the input processing blocks, including:

introducing, via a first delay stage (121), relative delays between signals that are a multiple of the sampling period of the signal processing stage; and
introducing, via a second delay stage (122), relative delays between signals that are a fraction of the sampling period of the signal processing stage;

wherein:

the fractional delays of the second delay stage are implemented by each input processing block using a Finite Impulse Response, FIR, filter comprising a plurality of capacitive elements and a plurality of switches for controlling transfer of charge between the capacitive elements;
for each input processing block, an input of the second delay stage is coupled to an output of the first delay stage, such that the plurality of capacitive elements is charged based on the output of the first delay stage; and
performing the discrete time analog signal processing of the signals includes controlling sequencing of operation of the switches.

**Patentansprüche**

1. Ultraschallverarbeitungssystem, das Folgendes umfasst:

mehrere steuerbare Eingabeverarbeitungsblöcke (320), wovon jeder eine zeitdiskrete Analogsignalverarbeitungsstufe zum Verarbeiten eines oder mehrerer entsprechender Eingangssignale, die Ultraschallsignale darstellen, implementiert, wobei jeder Eingabeverarbeitungsblock Folgendes umfasst:

eine erste Verzögerungsstufe (121), die gesteuert werden kann, um relative Verzögerungen zwischen Signalen einzuführen, wobei die relativen Verzögerungen ein Vielfaches der Abtastperiode der Signalverarbeitungsstufe sind; und
eine zweite Verzögerungsstufe (122), die gesteuert werden kann, um relative Verzögerungen zwischen Signalen einzuführen, wobei die relativen Verzögerungen ein Bruchteil der Abtastperiode der Signalverarbeitungsstufe sind;

wobei für jeden Eingabeverarbeitungsblock:

die bruchteiligen Verzögerungen der zweiten Verzögerungsstufe unter Verwendung eines Filters mit endlicher Impulsantwort (FIR-Filter), das mehrere kapazitive Elemente und mehrere Schalter zum Steuern der Ladungsübertragung zwischen den kapazitiven Elementen umfasst, implementiert werden;
ein Eingang der zweiten Verzögerungsstufe an einen Ausgang der ersten Verzögerungsstufe derart gekoppelt ist, dass die mehreren kapazitiven Elemente basierend auf der Ausgabe der ersten Verzögerungsstufe geladen werden; und
Eigenschaften der FIR-Filter entsprechend der Sequenzierung des Betriebs der Schalter gesteuert werden können.

2. Ultraschallverarbeitungssystem nach Anspruch 1, das ferner eines oder mehrere der folgenden Elemente umfasst:

a) mehrere Ultraschallelemente (115), die jeweils an einen entsprechenden Eingabeverarbeitungsblock gekoppelt sind, um ein Signal bereitzustellen, das ein Ultraschallsignal darstellt, das an dem Ultraschallelement empfangen worden ist;
b) mehrere Verstärker (310), die jeweils an einen Eingang eines entsprechenden Eingabeverarbeitungsblocks (320) gekoppelt sind;
c) einen Analog/Digital-Umsetzer (340), der an einen Ausgang jeder der Verarbeitungsstufen gekoppelt ist;
d) mehrere steuerbare Ausgabeverarbeitungsblöcke zum Erzeugen eines entsprechenden Signals für die Emission als ein Ultraschallsignal, wobei jeder Block eine zeitdiskrete Analogsignalverarbeitungsstufe implementiert, und gesteuert werden kann, um eine relative Verzögerung zwischen Signalen einzuführen, die ein Bruchteil der Abtastperiode der Signalverarbeitungsstufe sind.

3. Ultraschallverarbeitungssystem nach Anspruch 2, das ferner eine Steuereinrichtung (130, 330) umfasst, die an die Eingabeverarbeitungsblöcke gekoppelt sind, um den Betrieb des einen oder mehrerer der Eingabeverarbeitungsblöcke (320) zu steuern.

4. Ultraschallverarbeitungssystem nach Anspruch 1, das ferner Folgendes umfasst: eine Kombinationsschaltungsanordnung (123, 323) zum Kombinieren mehrerer Ausgaben der Eingabeverarbeitungsblöcke, um ein kombiniertes Signal zu bilden.

5. Ultraschallverarbeitungssystem nach Anspruch 4, wobei einer oder mehrere der folgenden Punkte zutreffen:

   a) das System umfasst ferner einen Analog/Digital-Umsetzer (ADC), der an einen Ausgang der Kombinationsschaltungsanordnung gekoppelt ist;
   b) die Sonde ist mit einer Basiseinheit durch eine Kommunikationsverbindung verbunden, die dafür geeignet ist, die kombinierten Signale an die Basiseinheit weiterzugeben.

6. Ultraschallsystem nach Anspruch 4, wobei sich die Eingabeverarbeitungsblöcke (320) und die Kombinationsschaltungsanordnung (123, 323) in einer tragbaren Sonde des System befinden.

7. Ultraschallsystem nach Anspruch 1, das ferner eine Steuereinrichtung (130, 330) umfasst, die an die Eingabeverarbeitungsblöcke gekoppelt ist, um die Eingabeverarbeitungsblöcke wiederholt neu zu konfigurieren.

8. Ultraschallsystem nach Anspruch 7, das ferner Folgendes umfasst: eine Kombinationsschaltungsanordnung (123, 323) zum Kombinieren mehrerer Ausgaben der Eingabeverarbeitungsblöcke, um ein kombiniertes Signal zu bilden, wobei die Steuereinrichtung (130, 330) auf die kombinierten Signale reagiert, um sich an Signalerfassungseigenschaften der Eingabesignale anzupassen.

9. Ultraschallsystem nach Anspruch 1, wobei jeder Eingabeverarbeitungsblock (320) Folgendes umfasst:

   mehrere passive Signalskalierungsschaltungen, die jeweils zum Annehmen eines analogen Eingangssignalwerts und eines digitalen Skalierungssteuerungswerts, der einen Skalierungsfaktor darstellt, und zum Speichern einer analogen Darstellung eines skalierten Signalwertes, der gleich einem Produkt des angenommenen Signalwerts und des Skalierungsfaktors ist, in einer Ausgabestufe für die Skalierungsschaltung, ausgelegt sind.

10. Ultraschallsystem nach Anspruch 9, wobei jede Signalskalierungsschaltung mehrere schaltbare, miteinander verbundene kapazitive Elemente umfasst.

11. Ultraschallsystem nach Anspruch 10, wobei im Betrieb der Skalierungsschaltung, der skalierte Signalwert in aufeinanderfolgenden Phasen gebildet wird, wobei jede Phase einer Konfiguration der schaltbaren Verbindung von kapazitiven Elementen zugeordnet ist, die Ladungsteilung unter miteinander verbundenen Kondensatoren erlaubt, und mindestens eines der kapazitiven Elemente entsprechend dem digitalen Skalierungssteuerungswert konfiguriert ist.

12. Verfahren zum Verarbeiten von durch Ultraschall erzeugten Signalen, das Folgendes umfasst:

   Annehmen mehrerer von mehreren durch Ultraschall erzeugten Signale;
   Verarbeiten der Signale in mehreren steuerbaren Eingabeverarbeitungsblöcken (320), die das Durchführen einer zeitdiskreten Analogsignalverarbeitung der Signale enthält;
   Steuern der Eingabeverarbeitungsblöcke, das Folgendes enthält:

   Einführen von relativen Verzögerungen zwischen Signalen, die einem Vielfachen der Abtastperiode der Signalverarbeitungsstufe entsprechen, über eine erste Verzögerungsstufe (121); und
   Einführen von relativen Verzögerungen zwischen Signalen, die einem Bruchteil der Abtastperiode der Signalverarbeitungsstufe entsprechen, über eine zweite Verzögerungsstufe (122);

   wobei:

   die bruchteiligen Verzögerungen der zweiten Verzögerungsstufe durch jeden Eingabeverarbeitungsblock unter Verwendung eines Filters mit endlicher Impulsantwort (FIR-Filter), das mehrere kapazitive Elemente und mehrere Schalter zum Steuern der Ladungsübertragung zwischen den kapazitiven Elementen umfasst, implementiert werden;
   für jeden Eingabeverarbeitungsblock ein Eingang der zweiten Verzögerungsstufe an einen Ausgang der ersten Verzögerungsstufe derart gekoppelt ist, dass die mehreren kapazitiven Elemente basierend auf der Ausgabe der ersten Verzögerungsstufe geladen werden; und

das Durchführen der zeitdiskreten Analogsignalverarbeitung der Signale das Steuern der Sequenzierung des Betriebs der Schalter enthält.

**Revendications**

1. Système de traitement par ultrasons comprenant :

une pluralité de blocs de traitement d'entrée pouvant être commandés (320), chacun mettant en œuvre un étage de traitement de signal analogique temporel distinct pour traiter un ou plusieurs signaux d'entrée correspondants représentant des signaux ultrasonores, dans lequel chaque bloc de traitement d'entrée comprend :

un premier étage de retards (121), pouvant être commandé pour introduire des retards relatifs entre des signaux, dans lequel les retards relatifs sont un multiple de la période d'échantillonnage de l'étage de traitement de signal ; et

un second étage de retards (122), pouvant être commandé pour introduire des retards relatifs entre des signaux, dans lequel les retards relatifs sont une fraction de la période d'échantillonnage de l'étage de traitement de signal ;

dans lequel, pour chaque bloc de traitement d'entrée :

les retards fractionnels du second étage de retards sont mis en œuvre en utilisant un filtre à réponse impulsionnelle finie (FIR) comprenant une pluralité d'éléments capacitifs et une pluralité de commutateurs pour commander un transfert de charge entre les éléments capacitifs ;

une entrée du second étage de retards est couplée à une sortie du premier étage de retards, de telle sorte que la pluralité d'éléments capacitifs est chargée sur la base de la sortie du premier étage de retards ; et

les caractéristiques du filtre FIR peuvent être commandées en fonction du séquençage de fonctionnement des commutateurs.

2. Système de traitement par ultrasons selon la revendication 1, comprenant en outre un ou plusieurs parmi :

a) une pluralité d'éléments ultrasonores (115), couplés chacun pour fournir un signal représentant un signal ultrasonore reçu au niveau de l'élément ultrasonore à un bloc de traitement d'entrée correspondant ;

b) une pluralité d'amplificateurs (310), couplés chacun à une entrée d'un bloc de traitement d'entrée correspondant (320) ;

c) un convertisseur analogique-numérique (340) couplé à une sortie de chacun des étages de traitement ;

d) une pluralité de blocs de traitement de sortie pouvant être commandés pour générer un signal correspondant pour une émission sous la forme d'un signal ultrasonore, chaque bloc mettant en œuvre un étage de traitement de signal analogique temporel distinct, et pouvant être commandé pour introduire un retard relatif entre des signaux qui sont une fraction de la période d'échantillonnage de l'étage de traitement de signal.

3. Système de traitement par ultrasons selon la revendication 2, comprenant en outre un dispositif de commande (130, 330) couplé aux blocs de traitement d'entrée pour commander le fonctionnement d'un ou plusieurs des blocs de traitement d'entrée (320).

4. Système de traitement par ultrasons selon la revendication 1, comprenant en outre : des circuits en combinaison (123, 323) pour combiner de multiples sorties des blocs de traitement d'entrée pour former un signal combiné.

5. Système de traitement par ultrasons selon la revendication 4, dans lequel un ou plusieurs des points suivants s'applique(nt) :

a) le système comprend en outre un convertisseur analogique-numérique, CAN, couplé à une sortie des circuits en combinaison ;

b) la sonde est reliée à une unité de base par une liaison de communication appropriée pour transmettre les signaux combinés à l'unité de base.

6. Système à ultrasons selon la revendication 4, dans lequel les blocs de traitement d'entrée (320) et les circuits en combinaison (123, 323) sont à l'intérieur d'une sonde portable du système.

7. Système à ultrasons selon la revendication 1, comprenant en outre un dispositif de commande (130, 330) couplé aux blocs de traitement d'entrée pour reconfigurer de manière répétée les blocs de traitement d'entrée.

8. Système à ultrasons selon la revendication 7, comprenant en outre : des circuits en combinaison (123, 323) pour combiner de multiples sorties des blocs de traitement d'entrée afin de former un signal combiné dans lequel le dispositif de commande (130, 330) est sensible aux signaux combinés pour s'adapter à des caractéristiques d'acquisition de signal des signaux d'entrée.

9.  Système à ultrasons selon la revendication 1, dans lequel chaque bloc de traitement d'entrée (320) comprend :
une pluralité de circuits de mise à l'échelle de signal passifs chacun pour accepter une valeur de signal d'entrée analogique et une valeur de commande de mise à l'échelle numérique représentant un facteur de mise à l'échelle, et stocker une représentation analogique d'une valeur de signal mise à l'échelle égale à un produit de la valeur de signal acceptée et du facteur de mise à l'échelle dans un étage de sortie pour le circuit de mise à l'échelle.

10. Système à ultrasons selon la revendication 9, dans lequel chaque circuit de mise à l'échelle de signal comprend une pluralité d'éléments capacitifs interconnectés de manière commutable.

11. Système à ultrasons selon la revendication 10, dans lequel lors du fonctionnement du circuit de mise à l'échelle, la valeur de signal mise à l'échelle est formée dans une succession de phases, chaque phase étant associée à une configuration de l'interconnexion commutable d'éléments capacitifs permettant un partage de charge entre des condensateurs interconnectés, au moins l'un des éléments capacitifs étant configuré conformément à la valeur de commande de mise à l'échelle numérique.

12. Procédé de traitement de signaux générés par ultrasons comprenant les étapes consistant à :

accepter une pluralité d'une pluralité de signaux générés par ultrasons ;
traiter les signaux dans une pluralité de blocs de traitement d'entrée pouvant être commandés (320), y compris réaliser un traitement de signal analogique temporel distinct des signaux ;
commander les blocs de traitement d'entrée, y compris :

introduire, via un premier étage de retards (121), des retards relatifs entre des signaux qui sont un multiple de la période d'échantillonnage de l'étage de traitement de signal ; et
introduire, via un second étage de retards (122), des retards relatifs entre des signaux qui sont une fraction de la période d'échantillonnage de l'étage de traitement de signal ;

dans lequel :

les retards fractionnels du second étage de retards sont mis en œuvre par chaque bloc de traitement d'entrée en utilisant un filtre à réponse impulsionnelle finie (FIR) comprenant une pluralité d'éléments capacitifs et une pluralité de commutateurs pour commander un transfert de charge entre les éléments capacitifs ;
pour chaque bloc de traitement d'entrée, une entrée du second étage de retards est couplée à une sortie du premier étage de retards, de telle sorte que la pluralité d'éléments capacitifs est chargée sur la base de la sortie du premier étage de retards ; et
la réalisation du traitement de signal analogique temporel distinct des signaux comprend une commande du séquençage de fonctionnement des commutateurs.

FIG. 1

FIG. 2

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0217298 A1
- US 4173007 A
- US 5676147 A
- US 6193659 B1
- US 5469851 A
- US 20100207644 A1

### Non-patent literature cited in the description

- Development of a Real-Time, High-Frequency Ultrasound Digital Beamformer for High-Frequency Linear Array Transducers. **CHANG-HONG HU et al.** IEEE Transactions on Ultrasonics, Ferroelectrics and Frequency control. IEEE, 2006, vol. 53